# EUROPEAN PATENT APPLICATION

(11) **EP 0 814 157 A2**
(43) Date of publication of application: **29.12.1997**
(21) Application number: 97304249.2
(22) Date of filing: 17.06.1997
(51) Int. Cl.: C12N 15/12, C07K 14/47, C12Q 1/68, C07K 16/18, G01N 33/566

(54) **Diagnostic marker for variants of presenilin genes associated with Alzheimers Disease and Familial Adult Onset Alzheimers Disease**

(30) Priority: 18.06.1996 US 19991 P
(71) Applicant: SMITHKLINE BEECHAM CORPORATION, Philadelphia Pennsylvania 19103 (US); WASHINGTON UNIVERSITY, St. Louis, MO 63130 (US); UNIVERSITY OF SOUTH FLORIDA, Tampa, FL 33620-6250 (US)
(72) Inventor: Barton, Amanda J. L., SmithKline Beecham Pharm., Harlow, Essex CM19 5AD (GB); Goate, Alison M., St Louis, MO 63130 (US); Hardy, John, University of South Florida, Tamp, Florida 33620 (US)
(74) Representative: Thompson, Clive Beresford

(57) **Abstract**

Methods are described for detecting the presence or absence of a four amino acid motif (VRXQ) in expressed proteins that arise from aberrant alternative splicing ofpre-mRNA in genes associated with normal neurological function which are useful for detecting neurodegenerative disease. The presence of these variants suggest that mutational events in these genes have occurred. Methods to measure the levels of gene expression of such genes to detect neurodegenerative disease are provided. Nucleotide sequences and intron-exon junctional sequences of examples of this splicing variant and probes for detecting this variant which are useful as diagnostic reagents are also provided.

## Description

### BACKGROUND OF THE INVENTION

In eukaryotes, the initial transcription of genomic DNA into RNA proceeds in the nucleus and yields a contiguous full-length reverse complementary heteronuclear RNA (hnRNA) primary transcript. The hnRNA contains regions or contiguous blocks of nucleotide sequence that end up in the final mRNA (exons) interspersed between "intervening" nucleotide sequences (introns) that do not. In addition to adenylyl methylation and polyadenylation, these hnRNAs are extensively modified in a process referred to as RNA "splicing" wherein discontiguous exons are joined and the intervening intron precisely deleted as an RNA "lariat" from the final mature mRNA transcript (B. Rushkin et al. Cell 1984, 38:317; R.A. Padgett et al. Science 1984, 225:898). RNA splicing is a complex process involving large protein-RNA assemblies called spliceosomes that coordinate the concerted excision and ligation events to yield intron-free mRNAs (M.M. Konarska and P.A. Sharp Cell 1987, 49:763; R. Reid et al. Cell 1988, 53:949; T.A. Steitz Sci. Am. 1988, 258:56).

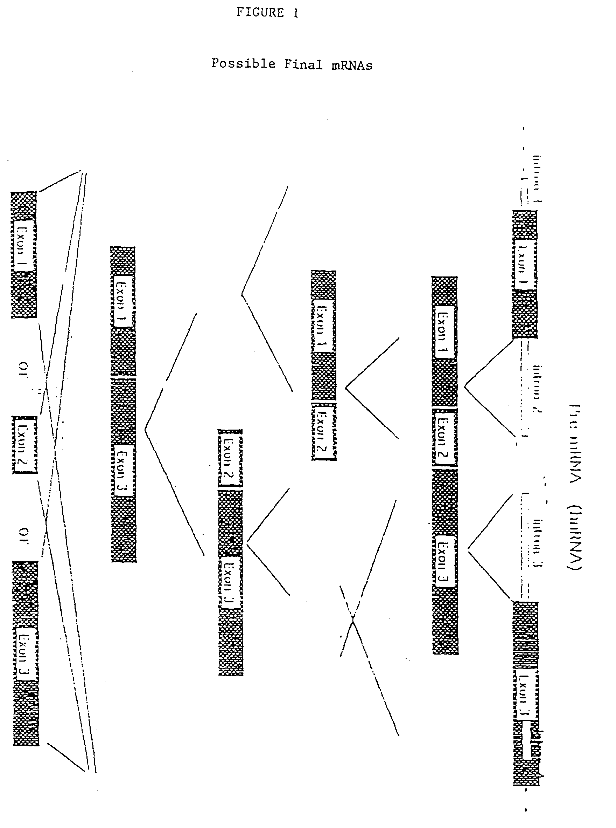

In normal RNA processing, the resultant mRNA reflects the linear sequence orientation of the exons in the hnRNA; however all exons do not end up in the final transcripts. Rather, several of the resultant mRNAs have only certain exons that result from "alternatively spliced" hnRNA, wherein discontiguous intron-exon junctions are spliced to bring for instance exon 1 and exon 4 into juxtaposition rather than exon 1 and exon 2. Therefore, several mRNAs may arise from one gene sequence or hnRNA. Not ail possible combinations of exons are normally represented in actual mRNA pools arising from one hnRNA as determined by mRNA, cDNA and protein analyses. As an example with three exons (Figure 1), while seven combinations are possible (exon1-exon2-exon3, exon1-exon2, exon1-exon3, exon2-exon3, exon1, exon2, or exon3) perhaps only two (exon1-exon2-exon3 and exon1-exon3) may actually result and be expressed at any appreciable level. These alternatively spliced transcripts are sometimes referred to as "variants". However, for purposes of this invention splice "variant" refers to heretofore unrepresented or expressed mRNAs arising from potential alternative splice sites that result from genomic mutation altering the structure of the hnRNA so that these splices now occur.

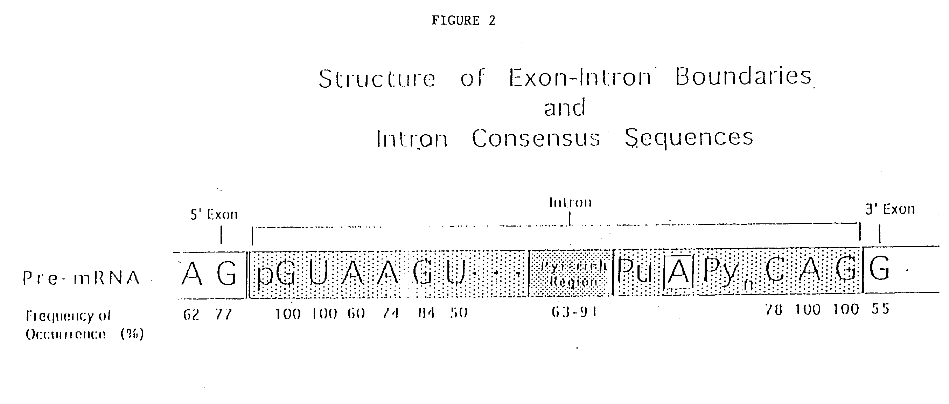

The location of splice sites in an hnRNA primary transcript can be determined by comparing the sequences of the corresponding genomic DNA with that of cDNA prepared by copying the corresponding mature mRNA. Any discontinuities between the genomic DNA and cDNA sequences mark the exon-intron boundaries. Such analyses of a number of different RNAs have defined moderately -short "consensus" sequences at the intron-exon boundaries in pre-mRNA and a tendency for a pyrimidine-rich region just upstream of the 3' splice junction (Figure 2). The only universally conserved nucleotides are the first two (GU) and last two (AG) in the intron (Figure 2), though there is a propensity for AG at the 5' exon termini and an initial G at the 3' exon. Only 30-40 nucleotides in the center portion of introns are necessary for efficient splicing. There is also a conserved A within the context of the pyrimidine rich region of the intron (Figure 2) (..PyrPyrPurAPyrnAG; where Pyr is a pyrimidine and Pur is a purine nucleotide) which is the branch point where the cleaved 5' exon-intron junction loops back to form the "lariat" splicing intermediate (Padgett et al. Science 1984, 225:898). Genetic point mutations that delete or alter these conserved intronic nucleotides (5' GU, 3' AG, or branch point A) would eliminate these splice junctions and prevent normal splicing yielding aberrantly truncated transcripts or transcripts where this exon is deleted and another downstream exon spliced in, that normally may not be spliced in. A final mechanism for splice variation occurs when several GU or AG dinucleotide motifs occur near consensus intron splice regions of 5' exon-intron or 3' intron-exon boundaries, respectively, such that the splicing system may sometimes not correctly distinguish the correct splice site resulting in alternate protein product some of which may be non-functional or aberrant.

Multiple examples of splice variations exist, many of which are associated with diseases or related disorders. Previous genetic linkage studies have shown a G to A mutation at the 3' splice junction of exon 8 of the gene encoding lysosomal acid lipase. Defects in this gene are associated with cholesterol ester storage disease that result in premature artherosclerosis, hepatomegaly, and elevated LDL cholesterol (U. Seedorf et al. Arterioscler. Throb. Vasc. Biol. 1995, 15: 773-778). Two mutations at the exon 1/intron l boundary altered the hepatic specific splicing of the human hydroxymethylbilane synthase gene (third enzyme in heme biosynthetic pathway) and resulted in an enzyme with half-normal activity (K.H. Astrin Human Mutat. 1994, 4:243-252). Deficiency of this enzyme activity eventually results in acute intermittent porphyria (AIP), an autosomal dominant inborn error of metabolism in which life-threatening attacks are precipitated by ecogenetic factors. Molecular cloning of cDNA and genomic DNA have provided probes allowing presymptomatic detection of these gene defects. In Menke's disease, a point mutation at the -2 exonic position of a splice donor site in the middle of the gene causes exon-skipping and activation of a cryptic splice acceptor site (S.G. Kaler et al. Nat. Genet. 1994, 8:195-202). Exon skipping of the entire exon 19 results from a G to A point mutation at the 5' donor site of intron 19 in muscle phosphofructokinase deficiency (T. Hamaguchi Biochem. Biophys. Res. Comm. 1994, 202:444-449). Aberrant RNA splicing from a splice site mutant in the interleukin-2 receptor gamma (gIL2-R) gene results in the generation ot an abundant non-functional gIL2-R containing a small intronic insertion and a second mutant form with 5-fold lower affinity (J.P. DiSanto et al. Proc. Natl. Acad. Sci. 1994, 91:9466-9470). These isoforms produce an atypical form of an X chromosome-linked severe combined immunodeficiency disease.

The presence of splice variants can be used as diagnostic markers of diseases associated with genetic mutations. For example, the expression of the exon 6 splice variant (v6) of the cell adhesion molecule CD44 is correlated with the expression of the tumor suppressor gene p53. Both have been shown to be markers of tumor progression in colorectal cancer (J.W. Mulder et al. Gut 1995, 36:76-80; Y. Matsumura Lancet 1992, 340:1053-1058). Asymptomatic carriers of the acute intermittent porphyria were identified by identification of a mutant allele containing a CG to CT transversion at the exonl/intron 1 boundary via in vitro amplification of DNA followed by hybridization of the target sequence to allele-specific oligonucleotides.

Accordingly, splicing variants have been observed in several gene loci and several diseases. Identification of these variants has proven to be especially useful in diagnosis and detection of asymptomatic carriers.

### SUMMARY OF THE INVENTION

A novel insertional motif that arises from splice mutations or alternative utilization of cryptic or less preferred splice donor sites has now been identified. These splicing variations result in the in-frame insertion within a normal protein sequence of four amino acids, valine-arginine-X-glutamine (VRXQ), where X is a hydrophilic amino acid. This motif has been identified in splice variants of a receptor, an enzyme, and a putative channel protein, all of which are involved in normal neurological functioning. Identification of this motif allows for screening of genes and gene products for splice variations.

The polynucleotides and polypeptides of the present invention may be employed as research reagents and materials for discovery of treatments and diagnostics to human disease, as further discussed herein reiating to poiynucieotide assays, inter alia.

A preferred method for the detection of this motif in expressed proteins in vitro or in situ with the use of specific antisera, polyclonal or monoclonal antibodies is provided. A method for the detection of allele-specific genetic mutations using selected oligonucleotides with standard hybridization-based detection techniques is also provided. A method for diagnosing Alzheimer's Disease (herein "AD") by detecting differences in levels of transcripts having the VRXQ insertion or proteins encoded therefrom is further provided. A preferred embodiment of such method for detecting AD provides for the detection of Familial Adult Onset Alzheimer's Disease (herein "FAD").

### BRIEF DESCRIPTION OF THE FIGURES

Figure 1 is a schematic of potential alternative splicing with 3 exons and 4 introns.

Figure 2 is a schematic of the consensus exon-intron-exon structure and sequence.

Figure 3 provides the sequence of the VRSQ variant of the presenilin I gene.

Figure 4 provides PS-1 Oligonucleotide Probes.

Figure 5 provides tabulated results of quantification of the ISH signal for PS-1-long and PS-1-short mRNAs in human brain.

### DETAILED DESCRIPTION OF THE INVENTION

The presenilin 1 (herein "PS-1") gene encodes a neuropeptide predicted to be a classical seven transmembrane protein (Sherrington et al. Nature 1995, 375:754-760). Missense mutations within this gene have been found in several families exhibiting early-onset AD. Genomic analysis has revealed the intron-exon boundaries of the hnRNA. A common polymorphism located within the intron 3' to exon 9 was identified in early onset AD patients. This polymorphism also showed a strong association with the occurrence of typical late onset AD families. This particular mutation did not produce an alteration in the coding sequence but is typical of variations leading to alternatively spliced proteins.

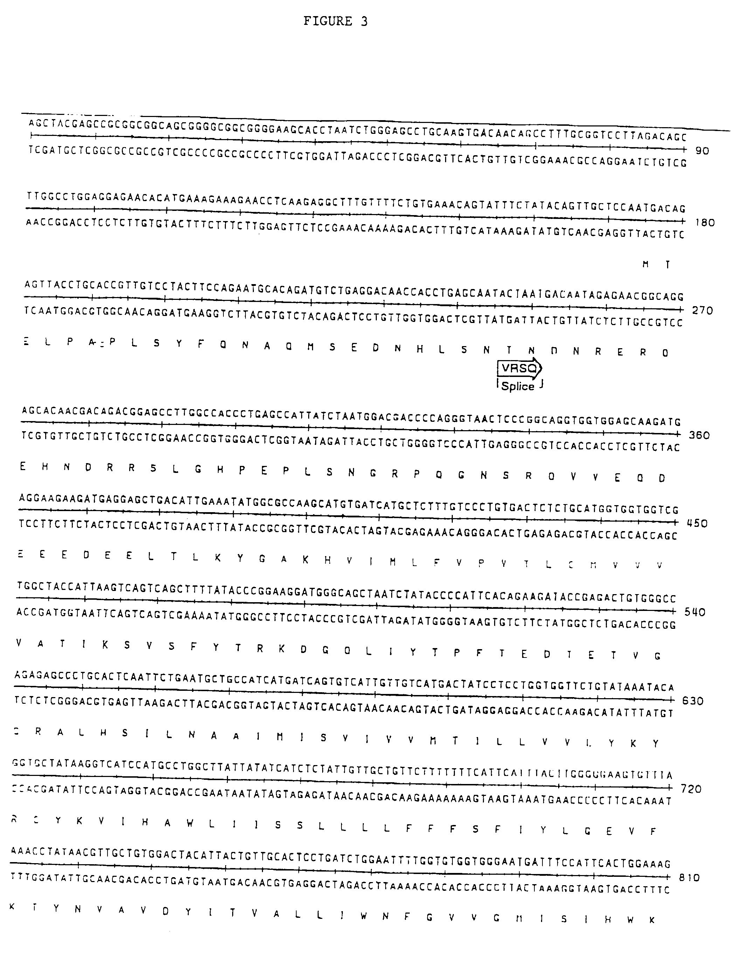

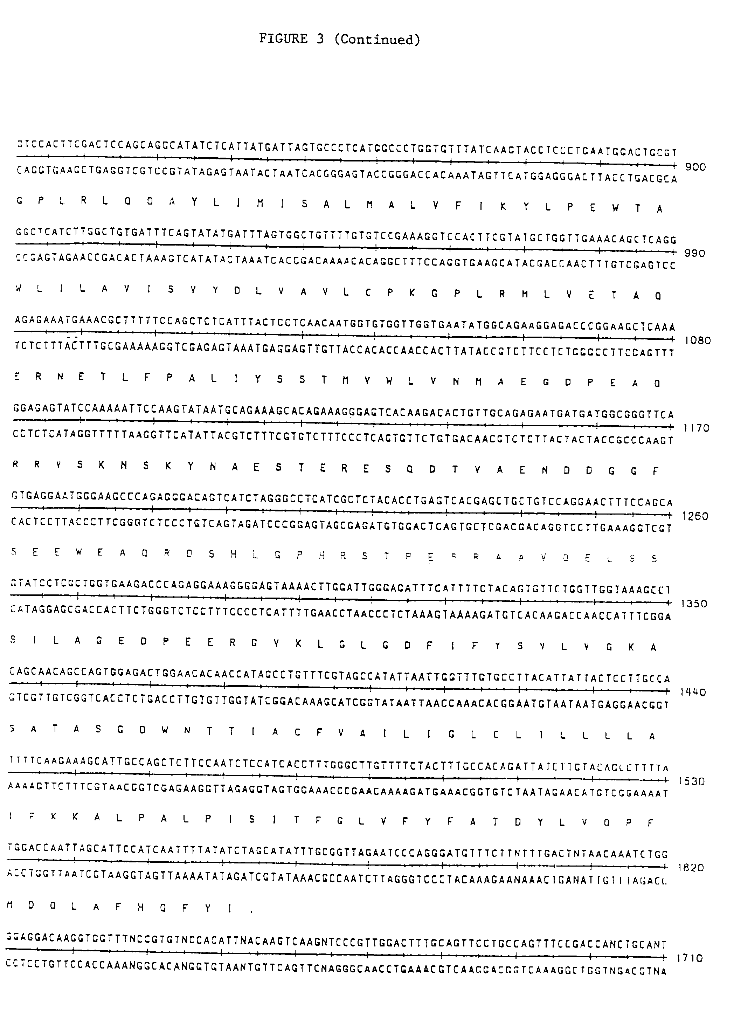

Other mutations within different introns of the PS-1 gene have been identified. These lead to alternatively spliced variants as well. One novel variant of the PS-1 protein isolated from a human cerebellar cDNA library contains a four amino acid insertion between codons 26 and 27 (VRSQ) (Figure 3). This variant arises from alternative use of a 5' exon dontor site in the exon 3/intron 3 boundary and results in the loss of some potential phosphorylation sites. A similar motif (VRXQ- where X is a hydrophilic amino acid) arising from aberrant splicing has also arisen due to alternative splicing in several other neurological proteins as well.

For example, the mRNA for tyrosine hydroxylase, the rate limiting enzyme in the synthesis of catecholomines, can undergo alternative splicing to produce several different isoforms (Kobayashi et al. J. Biochem. 1988, 103(6) 907-12; Lewis et al. Neuroscience 1993, 54(2) 477-92). The identified variants contain a 12 bp insertion encoding the sequence VRGQ. Isoforms containing the VRGQ insertion have also been found to exhibit alterations in phosphorylation by MAP kinase (Sutherland et al. Eur J Biochem. 1993, 217(2) 715-22). Furthermore, a tyrosine hydroxylase variant containing this insertion has been implicated in Parkinson's disease.

Another neuropeptide, gamma-Aminobutyric acid A (GABAA) receptor, undergoes alternative splicing to yield a multiplicity of transcripts (Whiting et al. P.N.A.S. 1990, 87(24) 9966-70; Lasham et al. Biochem. Soc. Trans. 1991, 19(1) 9S). GABA receptors are multisubunit ligand gated ion channels which mediate neuronal inhibition by GABAA and are composed of at least four subunit types (alpha, beta, gamma, and delta). The beta 4 subunit can undergo alternative splicing at two 5'-donor splice sites separated by 12 bp in the region that encodes the presumed intracellular loop between transmembrane domains M3 and M4. The insertion of the 12 bp sequence results in the addition of a VREQ motif (Bateson et al. J. Neurochem 1991, 56(4)1437-40).

In all three neurological proteins, the alternative splice site generates variants containing a specific motif (VRXQ) which appears to be intracellularly located and alters phosphorylation by various kinases.

In the present invention, a method for detecting the presence of the VRXQ motif in polyadenylated messenger RNA transcripts (polyA mRNA) and resultant expressed proteins, (where V is valine, R is arginine, X is any hydrophilic amino acid residue, and Q is glutamine), in cDNA resulting from these RNAs, and proteins, is provided. The VRXQ variant is referred to herein as "VRSQ variant". A method for quantitating such transcripts encoding and proteins having a VRXQ motif are also provided. Oligonucleotides having the anticodon sequences associated with the VRXQ motif having degenerate positions at the third base position of each codon can be used for the detection and quantitation of mRNA. Additionally, these oligonucleotides can be associated with codon sequences and used for the detection of cDNAs, and quantitation of the transcript from which the cDNA was derived. For example, codon and anticodon oligonucleotides for VRNQ comprise GU(N) AG(A/G) AA(C/U) CA(A/G) and the reverse complement. Hybridization of appropriate oligonucleotides can be detected and quantitated directly by procedures well known to those of skill in the art using radioactively or fluorescently labeled oligonucleotides. Indirect detection and quantitation procedures such as, but not limited to, biotinylated oligonucleotides/strepavidin-horseradish peroxidase, enhanced chemiluminescent detection, or fluorescently tagged strepavidins can also be performed.

Embodiments of the invention can be used to detect alterations in and make comparisons between structure and/or expression in of PS-1 variants in presumptive neurodegenerative disease, particularly neurodegenerative disease associated with head injury and AD, and more particularly chromosome 14 FAD. In a particularly preferred embodiment, probes and methods of the invention can be used to detect a reduction in the expression of PS-1 transcript encoding the VRSQ motif, shown by this invention to be a diagnostic marker for chromosome 14 FAD, since lowered levels are associated with chromosome 14 FAD. Preferred embodiments of the invention provide for comparisons between variants comprising the VRSQ region with those lacking it enabling the diagnosis of AD, particularly chromosome 14 FAD.

Further particularly preferred in this regard are polynucleotides encoding VRXQ variant variants, analogs, derivatives and fragments, and variants, analogs and derivatives of the fragments, which have the amino acid sequence of the VRXQ variant polypeptides of Figure 3 in which several, a few, 5 to 10, 1 to 5, 1 to 3, 2, 1 or no amino acid residues are substituted, deleted or added, in any combination. Also especially preferred among these are silent substitutions, additions and deletions, which do not alter the properties and activities of the VRXQ variant. Also especially preferred in this regard are conservative substitutions. Most highly preferred are polynucleotides encoding polypeptides having the amino acid sequence of Figure 3. without substitutions.

Further preferred embodiments of the invention are polynucleotides that are at least 70% identical to a polynucleotide encoding the VRXQ variant polypeptide having the amino acid sequence set out in Figure 3, and polynucleotides which are complementary to such polynucleotides. Alternatively, most highly preferred are polynucleotides that comprise a region that is at least 80% identical to a polynucleotide encoding the VRXQ variant polypeptide of the human cDNA of the deposited clone and polynucleotides complementary thereto. In this regard, polynucleotides at least 90% identical to the same are particularly preferred, and among these particularly preferred polynucleotides, those with at least 95% are especially preferred. Furthermore, those with at least 97% are highly preferred among those with at least 95%, and among these those with at least 98% and at least 99% are particularly highly preferred, with at least 99% being the more preferred.

Particularly preferred embodiments in this respect, moreover, are polynucleotides which encode polypeptides which retain substantially the same biological function or activity as the mature polypeptide encoded by the cDNA of Figure 3.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization wiii occur oniy if there is at least 95% and preferably at least 97% identity between the sequences.

As discussed additionally herein regarding polynucleotide assays of the invention, for instance, polynucleotides of the invention as discussed above, may be used as a hybridization probe for cDNA and genomic DNA to isolate full-length cDNAs and genomic clones encoding VRXQ variant polypeptides and to isolate cDNA and genomic clones of other genes that have a high sequence similarity to the human VRXQ variant polypeptide encoding gene. Such probes generally will comprise at least 15 bases. Preferably, such probes will have at least 30 bases and may have at least 50 bases. Particularly preferred probes will have at least 30 bases and will have 50 bases or less.

For example, the coding region of the VRXQ variant polynucleotide gene may be isolated by screening using the known DNA sequence to synthesize an oligonucleotide probe. A labeled oligonucleotide having a sequence complementary to that of a gene of the present invention is then used to screen a library of human cDNA, genomic DNA or mRNA to determine which members of the library the probe hybridizes to.

The polynucleotides and polypeptides of the present invention may be employed as research reagents and materials for discovery of treatments and diagnostics to human disease, as further discussed herein relating to polynucleotide assays, inter alia.

The polynucleotides may encode a polypeptide which is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may facilitate protein trafficking, may prolong or shorten protein half-life or may facilitate manipulation of a protein for assay or production, among other things. As generally is the case *in situ,* the additional amino acids may be processed away from the mature protein by cellular enzymes.

A precursor protein, having the mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In sum, a polynucleotide of the present invention may encode a mature protein, a mature protein plus a leader sequence (which may be referred to as a preprotein), a precursor of a mature protein having one or more prosequences which are not the leader sequences of a preprotein, or a preproprotein, which is a precursor to a proprotein, having a leader sequence and one or more prosequences, which generally are removed during processing steps that produce active and mature forms of the polypeptide.

### Polynucleotide assays

This invention is also related to the use of the VRXQ variant polynucleotides to detect complementary polynucleotides such as, for example, as a diagnostic reagent. Detection of a VRXQ variant associated with a dysfunction, particularly a neurological dysfunction, will provide a diagnostic tool that can add or define a diagnosis of a disease or susceptibility to a disease which results from under-expression over-expression or altered expression of VRXQ variant. Individuals carrying mutations in the expression gene level human VRXQ variant gene may be detected at the polynucleotide level by a variety of techniques. Nucleic acids for diagnosis may be obtained from a patient's cells, such as from blood, urine, saliva, tissue biopsy and autopsy material, particularly of nervous system origin. RNA, particularly mRNA, may be used directly for detection or may be amplified enzymatically by using PCR prior to analysis. PCR (Saiki et al., *Nature, 324:* 163-166 (1986)). cDNA may also be used in the same ways. As an example, PCR primers complementary to the nucleic acid encoding VRXQ variant can be used to identify and analyze VRXQ variant expression and mutations thereof. For example, deletions and insertions can be detected by a change in size of the amplified product in comparison to the normal genotype. Point mutations can be identified by hybridizing amplified DNA to radiolabeled VRXQ variant RNA or alternatively, radiolabeled VRXQ variant antisense DNA sequences. Perfectly matched sequences can be distinguished from mismatched duplexes by RNase A digestion or by differences in melting temperatures.

Sequence differences between a reference gene and genes having mutations also may be revealed by direct DNA sequencing. In addition, cloned DNA segments may be employed as probes to detect specific DNA segments. The sensitivity of such methods can be greatly enhanced by appropriate use of PCR or another amplification method. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alteration in electrophoretic mobility of DNA fragments in gels, with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers et al., *Science, 230:* 1242 (1985)).

Sequence changes at specific locations also may be revealed by nuclease protection assays, such as RNase and S1 protection or the chemical cleavage method (e.g., Cotton et al., *Proc. Natl. Acad Sci., USA, 85:* 4397-4401 (1985)).

Thus, the detection of a specific DNA sequence may be achieved by methods such as hybridization, RNase protection. chemical cleavage, direct DNA sequencing or the use of restriction enzymes, (e.g., restriction fragment length polymorphisms ("RFLP") and Southern blotting of genomic DNA.

In addition to more conventional gel-electrophoresis and DNA sequencing, mutations also can be detected by in situ analysis.

In accordance with a further aspect of the invention, there is provided a process for determining the presence of neurological disease, particularly AD, and neurodegenerative disease associated with head trauma, or a susceptibility to neurological disease, particularly AD, and neurodegenerative disease associated with head trauma. Thus, a mutation in VRXQ variant indicates a susceptibility to neuroiogicai disease, particularly AD, and neurodegenerative disease associated with head trauma, and the nucleic acid sequences described above may be employed in an assay for ascertaining such susceptibility. Thus, for example, the assay may be employed to determine a mutation in a human VRXQ variant protein as herein described, such as a deletion, truncation, insertion, frame shift, etc., with such mutation being indicative of a susceptibility to neurological disease, particularly AD, and neurodegenerative disease associated with head trauma.

A mutation may be ascertained for example, by a DNA sequencing assay. Tissue samples, including but not limited to blood samples are obtained from a human patient. The samples are processed by methods known in the art to capture the RNA. First strand cDNA is synthesized from the RNA samples by adding an oligonucleotide primer consisting of polythymidine residues which hybridize to the polyadenosine stretch present on the mRNA's. Reverse transcriptase and deoxynucleotides are added to allow synthesis of the first strand cDNA. Primer sequences are synthesized based on the DNA sequence of the DNA repair protein of the invention. The primer sequence is generally comprised of at least 15 consecutive bases, and may contain at least 30 or even 50 consecutive bases.

Individuals carrying mutations in the gene of the present invention may also be detected at the DNA level by a variety of techniques. The methods of the invention to detect and quantitate PS-1 polynucleotide sequence, PS-1 expression levels and gene expression products, particularly the immunological methods and methods using oligonucleotides, can be used with bodily tissues and fluids from individuals. Preferred bodily tissues and fluids useful with the methods of the invention include, but are not limited to, blood cells, plasma, skin cells, and brain cells, particularly neuronal, glial, and astrocyte cells. Further, nucleic acids for diagnosis may be obtained from a patient's cells, including but not limited to blood, urine, saliva, tissue biopsy and autopsy material.

The genomic DNA may be used directly for detection or may be amplified enzymatically by using PCR (Saiki *et al., Nature, 324*:163-166 (1986)) prior to analysis. RT-PCR can also be used to detect mutations. It is particularly preferred to used RT-PCR in conjunction with automated detection systems, such as, for example, GeneScan. RNA or cDNA may aiso be used for the same purpose, PCR or RT-PCR. As an example, PCR primers complementary to the nucleic acid encoding VRXQ variant can be used to identify and analyze mutations. For example, deletions and insertions can be detected by a change in size of the amplified product in comparison to the normal genotype. Point mutations can be identified by hybridizing amplified DNA to radiolabeled RNA or alternatively, radiolabeled antisense DNA sequences. Perfectly matched sequences can be distinguished from mismatched duplexes by RNase A digestion or by differences in melting temperatures. Oligonucleotide primers may be used for amplifying VRXQ variant cDNA isolated from a sample derived from a patient. The primers may be used to amplify the gene isolated from the patient such that the gene may then be subject to various techniques for elucidation of the DNA sequence. In this way, mutations in the DNA sequence may be diagnosed.

Sequence differences between the reference gene and genes having mutations may be revealed by the direct DNA sequencing method. In addition, cloned DNA segments may be employed as probes to detect specific DNA segments. The sensitivity of this method is greatly enhanced when combined with PCR. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers *et al., Science, 230*: 1242(1985)).

Sequence changes at specific locations may also be revealed by nuclease protection assays, such as RNase and S1 protection or the chemical cleavage method (e.g., Cotton *et al.,* PNAS, USA, 85:4397-4401 (1985)).

Thus, the detection of a specific DNA sequence and/or quantitation of the level of the sequence may be achieved by methods such as hybridization, RNase protection, chemical cleavage, direct DNA sequencing or the use of restriction enzymes, (e.g., Restriction Fragment Length Polymorphisms (RFLP)) and Southern blotting of genomic DNA. The invention provides a process for diagnosing, disease, particularly neurological disease, and most particularly AD, and neurodegenerative disease associated with head trauma, comprising determining from a sample derived from a patient a decreased level of expression of polynucleotide having the sequence of Figure 3. Decreased expression of polynucleotide can be measured using any on of the methods well known in the art for the quantation of polynucleotides, such as, for example, PCR, RT-PCR, RNase protection, Northern blotting and other hybridization methods.

In addition to more conventional gel-electrophoresis and DNA sequencing, mutations can also be detected by *in situ* analysis.

Fluorescence *in situ* hybridization (FISH) of a cDNA clone to a metaphase chromosomal spread can be used to provide a precise chromosomal location.

Once a sequence has been mapped to a precise chromosomal location, the physical position of the sequence on the chromosome can be correlated with genetic map data. Such data are found, for example, in V. McKusick, *Mendelian Inheritance in Man* (publicly available on line via computer). The relationship between genes and diseases that have been mapped to the same chromosomal region are then identified through linkage analysis.

Unless otherwise stated, transformation was performed as described in the method of Graham, F. and Van der Eb, A., *Virology, 52*:456-457 (1973).

### Chromosome assays

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

In certain preferred embodiments in this regard, the cDNA herein disclosed is used to clone genomic DNA of a VRXQ variant gene. This can be accomplished using a variety of well known techniques and libraries, which generally are available commercially. The genomic DNA the is used for *in situ* chromosome mapping using well known techniques for this purpose. Typically, in accordance with routine procedures for chromosome mapping, some trial and error may be necessary to identify a genomic probe that gives a good in situ hybridization signal.

In some cases, in addition, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include in situ hybridization, prescreening with labeled flow-sorted chromosomes and preselection by hybridization to construct chromosome specific-cDNA libraries.

Fluorescence in situ hybridization ("FISH") of a cDNA clone to a metaphase chromosomal spread can be used to provide a precise chromosomal location in one step. This technique can be used with cDNA as short as 50 or 60. For a review of this technique, see Verma et al., *HUMAN CHROMOSOMES: A MANUAL OF BASIC TECHNIQUES,* Pergamon Press, New York (1988).

Once a sequence has been mapped to a precise chromosomal location, the physical position of the sequence on the chromosome can be correlated with genetic map data. Such data are found, for example, in V. McKusick, *MENDELIAN INHERITANCE IN MAN* (publicly available on line via computer). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

### Polypeptide assays

The present invention also relates to a diagnostic assays such as quantitative and diagnostic assays for detecting levels of VRXQ variant protein in cells and tissues, including determination of normal and abnormal levels. Thus, for instance, a diagnostic assay in accordance with the invention for detecting over-expression of VRXQ variant protein compared to normal control tissue samples may be used to detect the presence of a tumor, for example. Assay techniques that can be used to determine levels of a protein, such as an VRXQ variant protein of the present invention, in a sample derived from a host are well-known to those of skill in the art. Such assay methods include radioimmunoassays, competitive-binding assays, Western Blot analysis and ELISA assays. Among these ELISAs frequently are preferred. An ELISA assay initially comprises preparing an antibody specific to VRXQ variant, preferably a monoclonal antibody. In addition a reporter antibody generally is prepared which binds to the monoclonal antibody. The reporter antibody is attached a detectable reagent such as radioactive, fluorescent or enzymatic reagent, in this example horseradish peroxidase enzyme.

To carry out an ELISA a sample is removed from a host and incubated on a solid support, e.g. a poiystyrene dish, that binds the proteins in the sample. Any free protein binding sites on the dish are then covered by incubating with a non-specific protein such as bovine serum albumin. Next, the monoclonal antibody is incubated in the dish during which time the monoclonal antibodies attach to any VRXQ variant proteins attached to the polystyrene dish. Unbound monoclonal antibody is washed out with buffer. The reporter antibody linked to horseradish peroxidase is placed in the dish resulting in binding of the reporter antibody to any monoclonal antibody bound to VRXQ variant. Unattached reporter antibody is then washed out. Reagents for peroxidase activity, including a colorimetric substrate are then added to the dish. Immobilized peroxidase, linked to VRXQ variant through the primary and secondary antibodies, produces a colored reaction product. The amount of color developed in a given time period indicates the amount of VRXQ variant protein present in the sample. Quantitative results typically are obtained by reference to a standard curve.

A competition assay may be employed wherein antibodies specific to VRXQ variant attached to a solid support and labeled VRXQ variant and a sample derived from the host are passed over the solid support and the amount of label detected attached to the solid support can be correlated to a quantity of VRXQ variant in the sample.

### Antibodies

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

Specific antibodies against the VRXQ motif can also be used for detection of the motif and quantitation of proteins having the motif. Various procedures known in the art may be used for the production of such antibodies.

For example, these antibodies can be obtained by direct injection of a polypeptide containing a VRXQ motif into an animal, preferably a nonhuman. The antibody so obtained will then bind to polypeptides containing this motif. Such antibodies can then be used to isolate and quantitate polypeptides containing this motif from tissues.

For preparation of monoclonal antibodies, any technique which provides antibodies produced by continuous cell line cultures can be used. Examples include the hybridoma technique (Kohler and Milstein, Nature 1975, 256:495-497), the trioma technique, the human B-cell hybridoma technique (Kozbor et al., Immunology Today 1983, 4:72), and the EBV-hybridoma technique to produce human monoclonal antibodies (Cole et al. in Monoclonal Antibodies and Cancer Therapy, Alan R. Liss, Inc., 1985, pp. 77-96).

Techniques described for the production of single chain antibodies (U.S. Patent 4,946,778) can be adapted to produce single chain antibodies to the immunogenic motif of this invention. Also, transgenic mice may be used to express humanized antibodies to polypeptides containing this motif.

Primary antibody-antigen reactions can be visualized and quantitated secondarily by standard enzyme-linked immunosorbent assay (ELISA) procedures. An ELISA assay initially comprises preparing an antibody specific to a VRXQ motif, preferably a monoclonal antibody. In addition a reporter antibody is prepared against the monoclonal antibody. To the reporter antibody is attached a detectable reagent such as horse radish peroxidase. A sample is then removed from a host and incubated on a solid support, e.g., a polystyrene dish, that binds the proteins in the sample. Any free protein binding sites on the dish are then covered by incubating with a non-specific protein like BSA. Next, the monoclonal antibody is incubated in the dish during which time the monoclonal antibodies attach to any proteins containing the VRXQ motif attached to the polystyrene dish. All unbound monoclonal antibody is washed out with buffer. The reporter antibody linked to horseradish peroxidase is then placed in the dish resulting in binding of the reporter antibody to any monoclonal antibody bound to proteins containing the VRXQ motif. Unattached reporter antibody is then washed out. Peroxidase substrates are then added to the dish and the amount of color developed in a given time period is a measurement of the amount of protein containing the VRXQ motif present in a given volume of patient sample when compared against a standard curve to detect and quantitate the protein. Examples of other detectable reagents which can be used include, but are not limited to, luciferase and fluorescently or radioactively tagged secondary antibodies. Specific populations of immune cells or chimeric cells (e.g., hybridomas) that express antibodies to VRXQ epitopes on their cell surfaces and respond by degranulation or release of cellular contents such as histamines that can be detected functionally or preloaded radiolabeled metals such as chromium are also useful.

The above-described antibodies may be employed to isolate or to identify clones expressing the polypeptide or purify the polypeptide of the present invention by attachment of the antibody to a solid support for isolation and/or purification by affinity chromatography.

### VRXQ variant binding molecules and assays

This invention also provides a method for identification of molecules, such as receptor molecules, that bind VRXQ variant. Genes encoding proteins that bind VRXQ variant, such as receptor proteins, can be identified by numerous methods known to those of skill in the art, for example, ligand panning and FACS sorting. Such methods are described in many laboratory manuals such as, for instance, Coligan et al., *Current Protocols in Immunology 1(2):* Chapter 5 (1991).

For instance, expression cloning may be employed for this purpose. To this end polyadenylated RNA is prepared from a cell responsive to VRXQ variant, a cDNA library is created from this RNA, the library is divided into pools and the pools are transfected individually into cells that are not responsive to VRXQ variant. The transfected cells then are exposed to labeled VRXQ variant. (VRXQ variant can be labeled by a variety of well-known techniques including standard methods of radioiodination or inclusion of a recognition site for a site-specific protein kinase.) Following exposure, the cells are fixed and binding of VRXQ variant is determined. These procedures conveniently are carried out on glass slides.

Pools are identified of cDNA that produced VRXQ variant-binding cells. Subpools are prepared from these positives, transfected into host cells and screened as described above. Using an iterative sub-pooling and re-screening process, one or more single clones that encode the putative binding molecule, such as a receptor molecule, can be isolated.

Alternatively a labeled ligand can be photoaffinity linked to a cell extract, such as a membrane or a membrane extract, prepared from cells that express a molecule that it binds, such as a receptor molecule. Cross-linked material is resolved by polyacrylamide gel electrophoresis ("PAGE") and exposed to X-ray film. The labeled complex containing the ligand-receptor can be excised, resolved into peptide fragments, and subjected to protein microsequencing. The amino acid sequence obtained from microsequencing can be used to design unique or degenerate oligonucleotide probes to screen cDNA libraries to identify genes encoding the putative receptor molecule.

Polypeptides of the invention also can be used to assess VRXQ variant binding capacity of VRXQ variant binding molecules, such as receptor molecules, in cells or in cell-free preparations.

The following examples are provided for illustrative purposes only and are not intended to limit the invention.

### EXAMPLES

### Example 1

A novel splice variant of the PS-1 gene described by Sherrington et al. Nature 1995, 375:754-760, was isolated from a human cerebellar and a human fibroblast library. In this novel splice variant there is a deletion of four amino acids at codons 26-27 (VRSQ). This arises from aiternative use of a 5' exon donor site in the exon3/intron 3 (-52 to 75 nt) boundary. The ...CAG/gta... boundary of the final Gln codon of exon 3 of the VRSQ motif provides a 5' exon AG donor site and GT intron consensus 5' boundary and use of this splice site results in the insertion of the 12-nts encoding the VRSQ motif. The upstream ...ACT/GTA... boundary of the Thr-Val codons provides the less preferred CT (AG preferred) 5' exonic boundary to the consensus GT 5' intronic boundary and splicing at this site would remove the VRSQ motif. Interestingly, in the PS-1 protein of Sherrington et al. Nature 1995, 375:754-760, this is the sole observed product and point mutations are interspersed elsewhere.

### Example 2

In the GABA receptor 4 subunit alternative splicing adds a VREQ motif (Bateson et al. J. Neurochem 1991, 56(4) 1437-40). A chicken genomic cDNA library was screened with chicken beta- 4' subunit cDNA at high stringency. Southern blot analysis, using cDNA sequence specific oligonucleotides as probes and subsequent restriction mapping allowed the identification of overlapping DNA fragments containing the coding regions of the beta-4 subunit gene. These fragments were subcloned into pBluescript and sequenced. Complete sequencing of one of the clones revealed the presence of 12 bp in the part encoding the intracellular loop (amino acid residues 335-338). Analysis of the beta-4 subunit gene reveals that the different transcripts encoding the two variants (absence or presence of 12bp loop) arise by the use of one of two 5'-donor splice sites (located in the intron immediately 3' of the 12 bp sequence).

### Example 3

The expression of two PS-1 mRNA transcripts, one containing (herein " PS-1-long") and one lacking the VSRQ motif (herein "PS-1-short"), in the brains of patients with early onset FAD was analyzed. In situ hybridization (ISH) was used to determine the qualitative and quantitative pattern of expression of PS-1 mRNA in the brains of early onset (presumptive chromosome 14-linked) FAD cases; comparisons with brains from patients with late onset AD and from normal individuals were made.

### In Situ Hybridization

PS-1 mRNA expression was examined in 4 neurologically normal control cases, 6 late onset AD cases and 3 early onset FAD cases. The late onset cases were thought to be of a sporadic nature as there was no evidence of family history and the mean age at death was 81.2 years (range: 79-84 years); they had a mean post mortem delay of 8.3 hours. The early onset FAD cases were presumed to be linked to chromosome 14 as they all had onset ages, family history, clinical presentations and histopathology typical of chromosome 14-linked FAD. For these the mean age at death was 45 years (range: 44-46 years) and the mean post mortem delay was 41.7 hours. Ail AD cases were diagnosed according to standard pathological criteria (Khachaturian, 1985, Archives of Neurology. 42:1097-1105). The controls had a mean age at death of 68.8 years (range: 57-85 years) and mean post mortem delay of 11.8 hours. The brain regions examined were the hippocampus, temporal cortex and frontal cortex (regions severely affected by AD pathology), the visual cortex (an area relatively unaffected, but which at the time of death may be in the early stages of the disease process) and the cerebellum (an area not affected by the classic pathology associated with AD and with no clinical involvement).

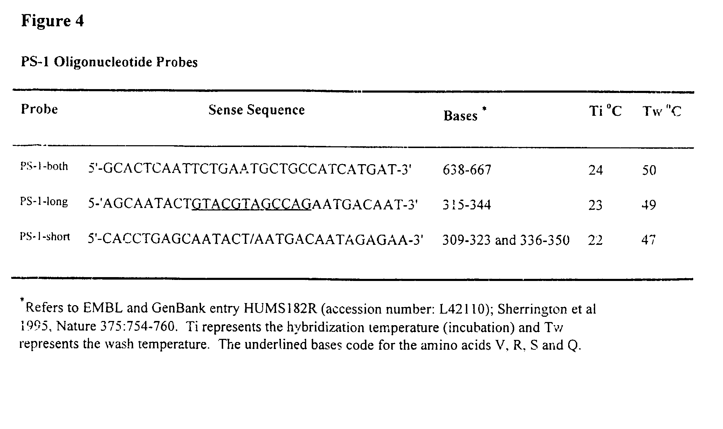

Three different oligoprobes were chosen and synthesized (Figure 4): one to detect PS-1-long, one to PS-1-short and one that recognizes both transcripts, PS-1-both. These probes are not predicted to detect the transcripts of presenilin-2, a closely related gene on chromosome 1 (Rogaev, et al., 1995, Nature 376:775-78).

The ISH methodology is well known in the art and has been described in detail elsewhere (Najlerahim et al., 1990, FEBS Letters 7:317-333). For the ISH analyses 10(m cryostat tissue sections were used. Probes were labelled at their 3' end with ³⁵S-dATP using the NEN DuPont 3' end labelling system. Hybridization and wash temperatures for the various probes are given in Figure 4. Hybridized sections were apposed to tritium-sensitive film for the generation of autoradiographs. Hybridization with the PS-1 probes in the sense orientation on adjacent sections were used to control for non-specific background. The signal on autoradiographic film was quantified using an image analyzer (Seescan®). A representative area over most of a tissue section was measured: for example, in the hippocampus the different subfields were not separately quantified. The background signal (sense strand hybridization) was subtracted from the antisense signal. Statistical analysis of the data was performed using the well known two-tailed Student's t-test.

### Northern Analysis

Northern analysis was carried out with the PS-1-both probe on a Northern blot (Clontech®, catalogue number: 7750-1) containing polyA+ mRNA from a number of different human brain regions. The probe was 3' end labelled with 32P-dATP using terminal transferase and hybridized under standard conditions (Clontech®, data sheet).

### Diagnostic Methods and Reagents for FAD

*In situ* hybridization using all three probes revealed that PS-1 mRNA was present in all of the brain regions examined. Hybridization with a sense strand control probe gave a very low background signal. In the cerebral cortex (three regions) a signal was detected in both the grey and white matter, often with a similar intensity. A diffuse rather than laminar pattern was observed in grey matter and in the hippocampus the different subfields were not readily delineated (although the dentate gyrus was sometimes visible). In the cerebellum, the granule cell layer contained the most labelling. These data are consistent with PS-1 mRNA expression in both neurons and glia.

Northern analysis confirmed that the PS-1-both oligoprobe detected a major transcript in human brain of the correct size for PS-1 mRNA (in accordance with the sequence data of Sherrington et al, 1995, Nature 375:754-760). A major band of approximately 3.4 kb was detected in all brain regions examined, indicating a wide distribution in brain for PS-1 mRNA. The observation of PS-1 mRNA in corpus cal!osum is consistent with the interpretation from the ISH data that PS-1 is expressed in glia.

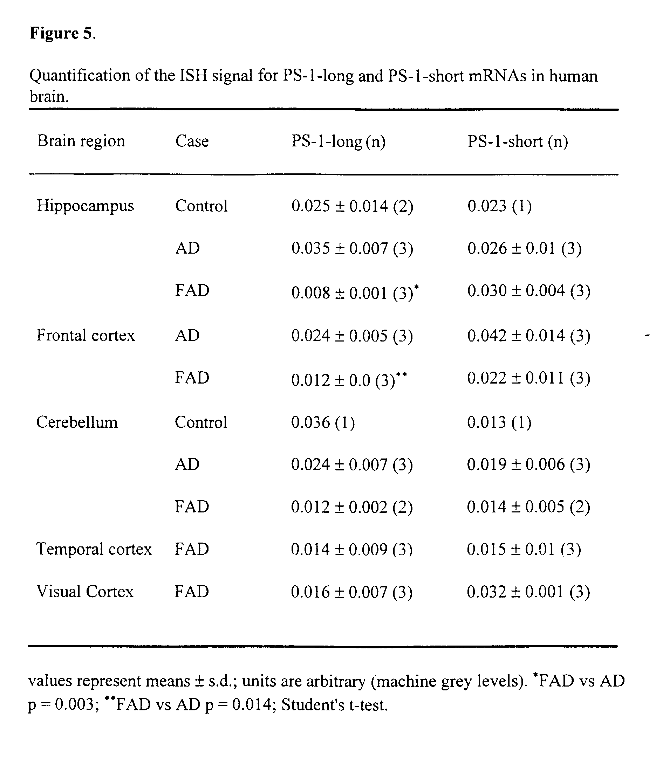

A similar anatomical pattern was seen by ISH, in each region, for both PS-1-long and PS-1-short transcripts. Nevertheless there appeared to be differences between the transcripts in their levels of expression according to brain region; for example PS-1-short was relatively less abundant in the cerebellum (Figure 5).

The hybridization pattern was similar for the controls, sporadic AD and FAD cases. Quantification of the autoradiographic film revealed a statistically significant reduction in the amount of PS-1-long mRNA in FAD hippocampus and frontal cortex compared with the sporadic AD cases (Figure 5; p = 0.003 and p = 0.014 respectively). In the cerebellum there was no significant difference between the controls, sporadic AD and FAD cases. The reduction in PS-1-long appears to be specific because there was no change in the level of expression of PS-1-short mRNA in any brain region investigated between the three different groups (Figure 5), which indicates reasonable data consistency.

## Claims

1. An isolated polynucleotide comprising a member selected from the group consisting of:
(a) a polynucleotide having at least one VRXQ motif and having at least a 70% identity to a polynucleotide encoding a polypeptide of Figure 3;
(b) a polynucleotide having at least one VRXQ motif and which is complementary to the polynucleotide of (a); and
(c) a polynucleotide having at least one VRXQ motif and comprising at least 15 bases of the polynucleotide of (a) or (b).

2. The polynucleotide of Claim 1 wherein the polynucleotide is DNA.

3. The polynucleotide of Claim 1 wherein the polynucleotide is RNA.

4. A polypeptide comprising the amino acid sequence of Figure 3.

5. A process for diagnosing a disease or a susceptibility to a disease related to expression of the polypeptide of claim 4 comprising:
determining a mutation in the nucleic acid sequence encoding said polypeptide.

6. A diagnostic process comprising:
analyzing for the presence of the polypeptide of claim 4 in a sample derived from a host.

7. A method for identifying compounds which bind to and activate or inhibit a receptor for the polypeptide of claim 4 comprising:
contacting a cell expressing on the surface thereof a receptor for the polypeptide, said receptor being associated with a second component capable of providing a detectable signal in response to the binding of a compound to said receptor, with a compound to be screened under conditions to permit binding to the receptor; and
determining whether the compound binds to and activates or inhibits the receptor by detecting the presence or absence of a signal generated from the interaction of the compound with the receptor.


SEQUENCE LISTING

(1) GENERAL INFORMATION

(i) APPLICANT
(A) NAME: SmithKline Beecham Corp
(B) STREET: Two New Horizons Court
(C) CITY: Brentford
(D) STATE OR PROVINCE: Middlesex
(E) COUNTRY: UK
(F) POSTAL CODE: TW8 9EP

(ii) TITLE OF THE INVENTION: A novel diagnostic marker
for variants of genes associated with neurological fu
nction

(iii) NUMBER OF SEQUENCES: 2

(iv) COMPUTER-READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(v) CURRENT APPLICATION DATA:
(A) APPLICATION NUMBER:

(2) INFORMATION FOR SEQ ID NO:1:

(i) SEQUENCE CHARACTERISTICS:
(A) LENGTH: 1750 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:1:

AGCTACGAGC CGCGGCGGCA GCGGGGCGGC GGGGAAGCAC CTAATCTGGG AGCCTGCAAG 60
TGACAACAGC CTTTGCGGTC CTTAGACAGC TTGGCCTGGA GGAGAACACA TGAAAGAAAG 120
AACCTCAAGA GGCTTTGTTT TCTGTGAAAC AGTATTTCTA TACAGTTCGT CCAATGACAG 180
AGTTACCTGC ACCGTTGTCC TACTTCCAGA ATGCACAGAT GTCTGAGGAC AACCACCTGA 240
GCAATACTAA TGACAATAGA GAACGGCAGG AGCACAACGA CAGACGGAGC CTTGGCCACC 300
CTGAGCCATT ATCTAATGGA CGACCCCAGG GTAACTCCCG GCAGGTGGTG GAGCAAGATG 360
AGGAAGAAGA TGAGGAGCTG ACATTGAAAT ATGGCGCCAA GCATGTGATC ATGCTCTTTG 420
TCCCTGTGAC TCTCTGCATG GTGGTGGTCG TGGCTACCAT TAAGTCAGTC AGCTTTTATA 480
CCCGGAAGGA TGGGCAGCTA ATCTATACCC CATTCACAGA AGATACCGAG ACTGTGGGCC 540
AGAGAGCCCT GCACTCAATT CTGAATGCTG CCATCATGAT CAGTGTCATT GTTGTCATGA 600
CTATCCTCCT GGTGGTTCTG TATAAATACA GGTGCTATAA GGTCATCCAT GCCTGGCTTA 660
TTATATCATC TCTATTGTTG CTGTTCTTTT TTTCATTCAT TTACTTGGGG GAAGTGTTTA 720
AAACCTATAA CGTTGCTGTG GACTACATTA CTGTTGCACT CCTGATCTGG AATTTTGGTG 780
TGGTGGGAAT GATTTCCATT CACTGGAAAG GTCCACTTCG ACTCCAGCAG GCATATCTCA 840
TTATGATTAG TGCCCTCATG GCCCTGGTGT TTATCAAGTA CCTCCCTGAA TGGACTGCGT 900
GGCTCATCTT GGCTGTGATT TCAGTATATG ATTTAGTGGC TGTTTTGTGT CCGAAAGGTC 960
CACTTCGTAT GCTGGTTGAA ACAGCTCAGG AGAGAAATGA AACGCTTTTT CCAGCTCTCA 1020
TTTACTCCTC AACAATGGTG TGGTTGGTGA ATATGGCAGA AGGAGACCCG GAAGCTCAAA 1080
GGAGAGTATC CAAAAATTCC AAGTATAATG CAGAAAGCAC AGAAAGGGAG TCACAAGACA 1140
CTGTTGCAGA GAATGATGAT GGCGGGTTCA GTGAGGAATG GGAAGCCCAG AGGGACAGTC 1200
ATCTAGGGCC TCATCGCTCT ACACCTGAGT CACGAGCTGC TGTCCAGGAA CTTTCCAGCA 1260
GTATCCTCGC TGGTGAAGAC CCAGAGGAAA GGGGAGTAAA ACTTGGATTG GGAGATTTCA 1320
TTTTCTACAG TGTTCTGGTT GGTAAAGCCT CAGCAACAGC CAGTGGAGAC TGGAACACAA 1380
CCATAGCCTG TTTCGTAGCC ATATTAATTG GTTTGTGCCT TACATTATTA CTCCTTGCCA 1440

TTTTCAAGAA AGCATTGCCA GCTCTTCCAA TCTCCATCAC CTTTGGGCTT GTTTTCTACT 1500
TTGCCACAGA TTATCTTGTA CAGCCTTTTA TGGACCAATT AGCATTCCAT CAATTTTATA 1560
TCTAGCATAT TTGCGGTTAG AATCCCAGGG ATGTTTCTTN TTTGACTNTA ACAAATCTGG 1620
GGAGGACAAG GTGGTTTNCC GTGTNCCACA TTNACAAGTC AAGNTCCCGT TGGACTTTGC 1680
AGTTCCTGCC AGTTTCCGAC CANCTGCANT TTNGGACTTG GAGGGGGNCC TAGGNAACGG 1740
TTTTGACCAA 1750

(2) INFORMATION FOR SEQ ID NO:2:

(i) SEQUENCE CHARACTERISTICS:
(A) LENGTH: 463 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:2:

Met Thr Glu Leu Pro Ala Pro Leu Ser Tyr Phe Gln Asn Ala Gln Met
1               5                   10                  15
Ser Glu Asp Asn His Leu Ser Asn Thr Asn Asp Asn Arg Glu Arg Gln
            20                  25                  30
Glu His Asn Asp Arg Arg Ser Leu Gly His Pro Glu Pro Leu Ser Asn
        35                  40                  45
Gly Arg Pro Gln Gly Asn Ser Arg Gln Val Val Glu Gln Asp Glu Glu
    50                  55                  60
Glu Asp Glu Glu Leu Thr Leu Lys Tyr Gly Ala Lys His Val Ile Met
65                  70                  75                  80
Leu Phe Val Pro Val Thr Leu Cys Met Val Val Val Val Ala Thr Ile
                85                  90                  95
Lys Ser Val Ser Phe Tyr Thr Arg Lys Asp Gly Gln Leu Ile Tyr Thr
            100                 105                 110
Pro Phe Thr Glu Asp Thr Glu Thr Val Gly Gln Arg Ala Leu His Ser
        115                 120                 125
Ile Leu Asn Ala Ala Ile Met Ile Ser Val Ile Val Val Met Thr Ile
    130                 135                 140
Leu Leu Val Val Leu Tyr Lys Tyr Arg Cys Tyr Lys Val Ile His Ala
145                 150                 155                 160
Trp Leu Ile Ile Ser Ser Leu Leu Leu Leu Phe Phe Phe Ser Phe Ile
                165                 170                 175
Tyr Leu Gly Glu Val Phe Lys Thr Tyr Asn Val Ala Val Asp Tyr Ile
            180                 185                 190
Thr Val Ala Leu Leu Ile Trp Asn Phe Gly Val Val Gly Met Ile Ser
        195                 200                 205
Ile His Trp Lys Gly Pro Leu Arg Leu Gln Gln Ala Tyr Leu Ile Met
    210                 215                 220
Ile Ser Ala Leu Met Ala Leu Val Phe Ile Lys Tyr Leu Pro Glu Trp
225                 230                 235                 240
Thr Ala Trp Leu Ile Leu Ala Val Ile Ser Val Tyr Asp Leu Val Ala
                245                 250                 255
Val Leu Cys Pro Lys Gly Pro Leu Arg Met Leu Val Glu Thr Ala Gln
            260                 265                 270
Glu Arg Asn Glu Thr Leu Phe Pro Ala Leu Ile Tyr Ser Ser Thr Met
        275                 280                 285
Val Trp Leu Val Asn Met Ala Glu Gly Asp Pro Glu Ala Gln Arg Arg
    290                 295                 300
Val Ser Lys Asn Ser Lys Tyr Asn Ala Glu Ser Thr Glu Arg Glu Ser
305                 310                 315                 320
Gln Asp Thr Val Ala Glu Asn Asp Asp Gly Gly Phe Ser Glu Glu Trp
                325                 330                 335
Glu Ala Gln Arg Asp Ser His Leu Gly Pro His Arg Ser Thr Pro Glu
            340                 345                 350
Ser Arg Ala Ala Val Gln Glu Leu Ser Ser Ser Ile Leu Ala Gly Glu
        355                 360                 365

Asp Pro Glu Glu Arg Gly Val Lys Leu Gly Leu Gly Asp Phe Ile Phe
    370                 375                 380
Tyr Ser Val Leu Val Gly Lys Ala Ser Ala Thr Ala Ser Gly Asp Trp
385                 390                 395                 400
Asn Thr Thr Ile Ala Cys Phe Val Ala Ile Leu Ile Gly Leu Cys Leu
                405                 410                 415
Thr Leu Leu Leu Leu Ala Ile Phe Lys Lys Ala Leu Pro Ala Leu Pro
            420                 425                 430
Ile Ser Ile Thr Phe Gly Leu Val Phe Tyr Phe Ala Thr Asp Tyr Leu
        435                 440                 445
Val Gln Pro Phe Met Asp Gln Leu Ala Phe His Gln Phe Tyr Ile
    450                 455                 460